(19)

Europäisches Patentamt

European Patent Office

Office européen des brevets

(11)  **EP 3 483 201 A1**

(12)  **EUROPEAN PATENT APPLICATION**

(43) Date of publication:
**15.05.2019  Bulletin 2019/20**

(21) Application number: **17201626.3**

(22) Date of filing: **14.11.2017**

(51) Int Cl.:
**C08G 83/00** (2006.01)  **C08G 65/22** (2006.01)
**C08G 65/26** (2006.01)  **C08G 65/326** (2006.01)
**A61K 47/34** (2017.01)  **C08G 63/08** (2006.01)
**C08G 63/85** (2006.01)  **C08G 63/91** (2006.01)
**C08G 65/334** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Freie Universität Berlin**
**14195 Berlin (DE)**

(72) Inventors:
• **HAAG, Rainer**
  **12209 Berlin (DE)**

• **MOHAMMADIFAR, Ehsan**
  **14129 Berlin (DE)**
• **FERRARO, Magda**
  **12163 Berlin (DE)**
• **ZABIHI, Fatemeh**
  **12247 Berlin (DE)**
• **ADELI, Mohsen**
  **12247 Berlin (DE)**

(74) Representative: **Maikowski & Ninnemann**
**Patentanwälte Partnerschaft mbB**
**Postfach 15 09 20**
**10671 Berlin (DE)**

(54)  **METHOD FOR MANUFACTURING A HYPERBRANCHED POLYESTER POLYOL DERIVATIVE**

(57)  The present invention relates to a method for manufacturing a hyperbranched polyester polyol derivative, comprising the following steps: a) reacting only glycidol and ε-caprolactone to obtain a hyperbranched polyester polyol in which caprolactone residues are randomly arranged; b) reacting the hyperbranched polyester polyol of step a) with a sulfation reagent to obtain a sulfated hyperbranched polyester polyol as hyperbranched polyester polyol derivative. Aspects of the invention relate to a hyperbranched polyester polyol derivative obtainable by this method as well as uses thereof.

FIG 6B

**Description**

The present invention relates to a method for manufacturing a hyperbranched polyester polyol derivative according to the preamble of claim 1, to a hyperbranched polyester polyol derivative that can be produced with this method according to the preamble of claim 7, to uses of such a hyperbranched polyester polyol derivative according to the preambles of claims 9, 11 and 15, to a medicament comprising such a hyperbranched polyester polyol derivative according to the preamble of claim 13, as well as to uses of an intermediary product according to the preambles of claims 14 and 15

Hyperbranched polyglycerols (hPGs) offer a variety of biomedical applications due to their unique physicochemical properties such as biocompatibility and multi-functionality. However, lack of biodegradability under physiological conditions hampers their *in vivo* applications. In prior art, different approaches have been described in order to enhance the biodegradability of linear, star-shaped and branched polyglycerols. However, many of those approaches are connected with severe drawbacks.

WO 2012/031245 A1 describes a composition comprising hyperbranched polyglycerol macromers, a cross-linker, a biodegradable component and a thermoresponsive component. This composition is manufactured by producing hyperbranched polyglycerol in a first step, adding methacrylate residues in a second step, preparing 2-hydroxyethyl methacrylate-poly(lactic acid) (HEMAPLA) in a third step, and producing a copolymer of the methacrylate-activated polyglycerol and HEMAPLA. The resulting compound has a star-shaped structure, i.e., it is no hyperbranched polymer, but only comprises a polar hyperbranched polyglycerol core onto which HEMAPLA residues are attached. The polyglycerol core of this compound is not biodegradable even after HEMAPLA addition.

US 2015/0037375 A1 describes a surfactant in the form of an oligoglycerol typically having 3 to 4 glycerol units. It is (chemically incorrect) also denoted as polyglycerol and has a star-shaped structure with grafted substituents. Other manufacturing methods described in this patent application lead to a linear polymer (due to the use of a protective group) or to a hydrophobic linear block copolymer comprising glycerol units.

US 2016/0331875 A1 describes a linear block copolymer made of caprolactone units and glycerol units. Due to its structure, no surface modification like sulfation of this linear block copolymer is possible.

Li and Li (Li, Z., & Li, J. (2013): Control of hyperbranched structure of polycaprolactone/poly (ethylene glycol) polyurethane block copolymers by glycerol and their hydrogels for potential cell delivery. The Journal of Physical Chemistry B, 117(47), 14763-14774) describe a complex manufacturing method for a polymer always comprising polyethylene glycol (PEG) segments, caprolactone segments and glycerol residues. The reaction necessitates hexamethylene diisocyanate (HDI) as coupling reagent. In doing so, the resulting polymer also always comprises HDI residues, wherein the PEG segments, caprolactone segments and glycerol residues are linked to each other by urethane linkages.

Zhou et al. (Zhou, J., Wang, W., Villarroya, S., Thurecht, K. J., & Howdle, S. M. (2008): Epoxy functionalised poly (ε-caprolactone): synthesis and application. Chemical Communications, (44), 5806-5808) describe an epoxy functionalization of ε-caprolactone, wherein the functionalized ε-caprolactone is further copolymerized with $CO_2$ or succinic anhydride. The resulting copolymer comprises polycarbonate residues or polysuccinate residues. In order to keep the glycidol ring intact during the initial functionalization step, lipase is used as catalyst.

It is an object of the present invention to provide a particularly simple manufacturing method for a pharmaceutically active compound that can also act as carrier for other pharmaceutically active compounds.

This object is achieved by a method for manufacturing a hyperbranched polyester polyol derivative having the features of claim 1. Such a manufacturing method is a two-step method and comprises the steps explained in the following. In a first step, glycidol is reacted with ε-caprolactone. A hyperbranched polyester polyol results in which caprolactone residues and glycerol residues are randomly arranged. The reaction is carried out as ring-opening polymerization so that the caprolactone residues in the resulting polymer are no longer circular but rather linear. They can also be denoted as hexanoyl residues.

In a second step, the hyperbranched polyester polyol of the first step is reacted with a sulfation reagent. A sulfated hyperbranched polyester polyol results that serves as hyperbranched polyester polyol derivative.

In an embodiment, the reaction is carried out as one-pot reaction. In an embodiment, it only consists of the precedingly explained steps (and optional purification steps). Thus, the hyperbranched polyester polyol derivative can be manufactured in an extremely simple manner. As will be explained below, it has anti-inflammatory properties, i.e., it is pharmaceutically active. Furthermore, it can be used as carrier for other pharmaceutically active substances.

Due to its simplicity, it is possible to perform this manufacturing method in technically largescale so that it is suited to produce significant amounts of the hyperbranched polyester polyol derivative.

It is neither necessary nor intended to copolymerize any other substances than glycidol and ε-caprolactone. The first step of the method specifically does not make use of any other substances than glycidol and ε-caprolactone to be polymerized. To be more precisely, no coupling reagents like HDI are used. In addition, no aromatic substances are used to be copolymerized for building up the hyperbranched polyester polyol derivative.

Due to the copolymerized caprolactone residues, the hyperbranched polyester polyol derivative comprises aliphatic hydrophobic segments. Together with hydrophilic glycerol residues, an overall amphiphilic structure results. It is particularly appropriate for transporting substances, in particular pharmaceutically active substances, in particular hydrophobic substances (whether pharmaceutically active or not).

The hyperbranched polyester polyol derivative has no core-shell structure, but rather shows a random structure having a statistical distribution of hydrophobic caprolactone residues within hydrophilic glycerol residues. A significant part of hydroxyl groups of the glycerol residues are sulfated.

It could be experimentally shown that the synthesized hyperbranched polyester polyol derivative is susceptible to enzymatic cleavage, but remains stable under neutral and acidic conditions. It showed high cellular uptake that was proven by laser scanning confocal microscopy (LSCM). MTT assays did not show a significant toxicity against HaCaT cells up to concentration of 1000 $\mu$g/ml. Thus, the hyperbranched polyester polyol derivative showed good biodegradability and biocompatibility. Furthermore, it was able to form nanoparticles in aqueous solutions and to load hydrophobic guest molecules.

In an embodiment, the first step of the method is carried out in presence of a catalyst. It turned out that a Lewis acid such as a tin compounds acting as Lewis acid is a particularly appropriate catalyst for this reaction step. In an embodiment, tin(II) 2-ethylhexanoate (tin octoate, $Sn(Oct)_2$) is used as catalyst.

The reaction can be carried out under mild (ambient) conditions. In an embodiment, the reaction is carried out at a reaction temperature lying in a range of between 30 °C and 150 °C, in particular between 40 °C and 140 °C, in particular between 50 °C and 130 °C, in particular between 60 °C and 120 °C, in particular between 70 °C and 110 °C, in particular between 80 °C and 105 °C, in particular between 90 °C and 100 °C. Particular appropriate reaction temperatures are temperatures at or around 50 °C, at or around 90 °C, and at or around 120 °C.

In an embodiment, a molar ratio between glycidol and ε-caprolactone is between 1 to 1 and 10 to 1, in particular between 2 to 1 and 9 to 1, in particular between 3 to 1 and 8 to 1, in particular between 4 to 1 and 7 to 1, in particular between 5 to 1 and 6 to 1. Thus, it is always used at least as much glycidol as ε-caprolactone, but particularly more glycidol than ε-caprolactone. Particular appropriate molar ratios between glycidol and ε-caprolactone are molar ratios of 2 to 1 and 4 to 1.

Obviously, the molar ratio between glycidol and ε-caprolactone influences the structure of the resulting hyperbranched polyester polyol. In addition, the reaction temperature also influences the resulting structure. Particular appropriate combinations between molar ratios and reaction temperatures are a molar ratio of 2 to 1 and a temperature of 50 °C, 90 °C, or 120 °C as well as a molar ratio of 4 to 1 and a temperature of 50 °C, 90 °C, or 120 °C.

The sulfation reagent used in the second step of the method is, in an embodiment, a sulfur trioxide base complex (e.g., $SO_3$·Pyridine, or $SO_3$·triethylamine). Sulfur trioxide pyridine complex is particularly appropriate. The sulfation can be carried out as generally known in the art. It takes place at the free hydroxyl groups of the hyperbranched polyester polyol so that a sulfated hyperbranched polyester polyol results.

Appropriate reaction conditions for the sulfation step comprise a reaction temperature of 40 °C to 80 °C, in particular of 45 °C to 75 °C, in particular of 50 °C to 70 °C, in particular of 55 °C to 65 °C, in particular of 60 °C to 80 °C, and/or a reaction duration of 12 hours to 2 days, in particular of 1 day to 1.5 days. Particular appropriate reaction conditions are a reaction temperature of 55 °C to 65 °C (such as 60 °C) and a reaction duration of approximately one day.

In an aspect, the present invention also relates to a hyperbranched polyester polyol derivative that can be obtained by a method according to any of the preceding explanations. As already stated above, the concrete structure of such hyperbranched polyester polyol derivative cannot be exactly described a more concrete terms than by making reference to its manufacturing method since the reaction temperature and the molar ratio of the educts, i.e., of glycidol and of ε-caprolactone, influence the resulting molecular structure of the hyperbranched polyester polyol derivative.

In an embodiment, the hyperbranched polyester polyol derivative has a degree of sulfation of from 70 to 100 %, in particular of from 75 to 99 %, in particular of from 80 to 98 %, in particular of from 85 to 97 %, in particular of from 90 to 95 %. Thus, a significant amount of hydroxyl groups of the hyperbranched polyester polyol resulting from the first step of the method is sulfated and the second step of the method according to this embodiment.

In an embodiment, the hyperbranched polyester polyol derivative has a number average molecular weight lying in a range of between 25 and 75 kDa, in particular of between 30 and 70 kDa, in particular of between 35 and 65 kDa, in particular of between 40 and 60 kDa, in particular of between 45 and 55 kDa.

The hyperbranched polyester polyol derivative as described above has a very good anti-inflammatory activity. This will be shown in detail with respect to exemplary embodiments. Due to this activity, the use of such a hyperbranched polyester polyol derivative as drug is also claimed.

In an embodiment, the drug is a drug for inhibiting the complement system of an organism and/or for inhibiting L-selectin binding to its natural receptor. By such an inhibition, an anti-inflammatory effect is achieved. Thus, in an embodiment, the hyperbranched polyester polyol derivative is used as drug for treating an inflammatory disease.

In an aspect, the invention relates to a medical method comprising administering a hyperbranched polyester polyol derivative according to the above explanations or a drug comprising such a hyperbranched polyester polyol derivative to a person in need thereof. This medical method is in particular a method for treating an inflammatory condition of the person.

In an embodiment, the method is a method for inhibiting the complement system of an organism and/or for inhibiting L-selectin binding to its natural receptor.

In another embodiment, the hyperbranched polyester polyol derivative is used for targeting inflammatory tissue in vivo. It is possible to couple the hyperbranched polyester polyol derivative with a detectable probe so as to use the construct of hyperbranched polyester polyol derivative and probe for diagnosing inflammatory diseases, in particular chronic inflammatory diseases such as rheumatoid arthritis or psoriasis or acute inflammatory processes such as those occurring after an organ transplant. Suited detectable probes are fluorescence probes, contrast agents, magnetic agents etc.

In an aspect, the invention relates to a medical method for targeting inflammatory tissue in vivo, comprising administering a hyperbranched polyester polyol derivative according to the above explanations or a drug comprising such a hyperbranched polyester polyol derivative to a person in need thereof. Any of the embodiments described in the preceding section for an according use can, in an embodiment, also be applied to such a medical method.

In an embodiment, the invention relates to a drug comprising a hyperbranched polyester polyol derivative according to the preceding explanations as active ingredient. However, the hyperbranched polyester polyol derivative cannot be used only as active ingredient, but also as a carrier molecule for transporting other molecules to a desired site of action (such as inflammatory tissue). To give an example, skin penetration tests showed that the synthesized polymers penetrated into the skin and transported the hydrophobic guest molecules through the stratum corneum to deeper skin layers. The hyperbranched polyester polyols (hPPs) were degraded to smaller segments in the presence of skin lysate. This property makes them proper candidates for the intradermal drug delivery due to both a facilitated drug release and low accumulation inside the tissue. Therefore, the invention relates in an embodiment also to a drug comprising both a hyperbranched polyester polyol derivative and additionally a further pharmaceutically active substance. Thereby, the hyperbranched polyester polyol derivative acts as carrier for the further pharmaceutically active substance and is itself pharmaceutically active.

In an aspect, the invention relates to a medical method comprising administering such a hyperbranched polyester polyol derivative or a drug comprising such a hyperbranched polyester polyol derivative carrying in each case a pharmaceutically active substance, in particular a hydrophobic pharmaceutically active substance, to a person in need thereof. In an embodiment, administering is carried out as intradermal administering.

In an aspect, the invention relates to the use of a hyperbranched polyester polyol that can be obtained by a method according to the first step of the method according to the above explanations (i.e., a non-sulfated intermediate product) as carrier for a pharmaceutically active substance, in particular of a hydrophobic pharmaceutically active substance. Such intermediate product does not carry sulfate groups and is thus not pharmaceutically active itself. As will be outlined in connection to an exemplary embodiment, the intermediate product is nonetheless well appropriate to act as substance carrier.

In an aspect, the invention relates to a medical method comprising administering such an intermediate product or a drug comprising such an intermediate product carrying in each case a pharmaceutically active substance, in particular a hydrophobic pharmaceutically active substance, to a person in need thereof. In an embodiment, administering is carried out as intradermal administering.

In an aspect, the invention relates to the use of a hyperbranched polyester polyol derivative that can be obtained by a method according to the above explanations in intradermal delivery of a pharmaceutically active substance, in particular of a hydrophobic pharmaceutically active substance.

In an aspect, the invention relates to the use of a hyperbranched polyester polyol that can be obtained by a method according to the first step of the method according to the above explanations (i.e., a non-sulfated intermediate product) in intradermal delivery of a pharmaceutically active substance, in particular of a hydrophobic pharmaceutically active substance.

All embodiments explained in connection to the described manufacturing method can also be applied to the described hyperbranched polyester polyol derivative, to the described medicament, and to the described uses, and vice versa in each case.

Details of aspects of the present invention will be explained with respect to exemplary embodiment and accompanying Figures. In the Figures:

Figure 1 shows a schematic depiction of the synthesis of a random copolymer of glycidol and $\varepsilon$-caprolactone through ring opening polymerization;

Figure 2A shows an IR spectrum of hPP41-50 as exemplary embodiment of a hyperbranched polyester polyol;

Figure 2B shows a $^1$H NMR spectrum of hPP41-50 recorded in $D_2O$;

Figure 2C shows a $^{13}$C NMR spectrum of hPP41-50 recorded in $D_2O$;

Figure 2D shows a $^1$H-HSQC NMR spectrum of hPP41-50 recorded in $D_2O$;

Figure 3 shows an assignment of different structural units in inverse-gated $^{13}$C NMR (upper panel) and $^{13}$C DEPT (lower panel) spectra of hPP41-50 in $D_2O$;

Figure 4    shows scanning electron microscopy (SEM) images (in two scales) and DLS diagrams of hPPs synthesized at 50 °C (upper row) and 120 °C (lower row);

Figure 5A    shows a gel permeation chromatography (GPC) diagram of (i) pristine hPOGC41-50, its degradation products in presence of (ii) skin lysate after 12 hours and (iii) Novozyme after 3 days;

Figure 5B    shows cytotoxicity results obtained from a CCK8 assay upon incubation of HaCaT cells with hPP41-50 after 24 h;

Figure 6A    shows a schematic depiction of the synthetic pathway for the sulfation of terminal hydroxyl groups of a hyperbranched polyester polyol;

Figure 6B    shows a schematic depiction of a sulfated hyperbranched polyester polyol;

Figure 7    shows a $^1$H NMR spectra of hPP and its sulfated derivative both measured in $D_2O$;

Figure 8    shows a schematic depiction of the synthetic pathway of dye-conjugate polymer synthesis;

Figure 9    shows the results of a cell viability test on A549 cell line;

Figure 10    shows the results of a cell viability test on Caco-2 cell line;

Figure 11    shows the results of fluorescence-activated cell sorting of A549 cells and Caco-2 cells;

Figure 12    shows the results of an L-selectin inhibition tests;

Figure 13    shows the results of a blood clotting assay; and

Figure 14    shows the results of a complement activation test.

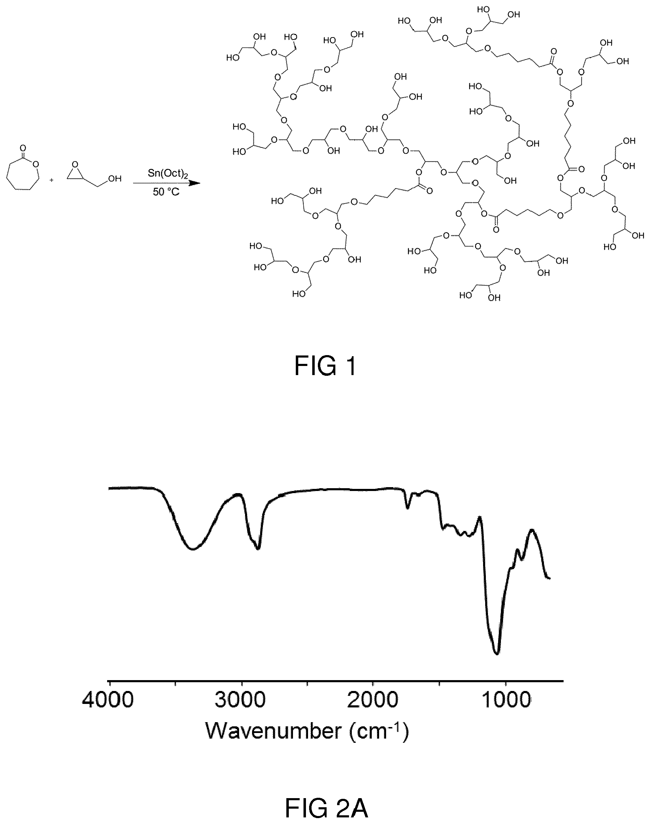

Figures 1 to 5B will be explained in connection to an exemplary embodiment relating to different non-sulfated hyperbranched polyester polyols, i.e., intermediate products resulting from the first step of the above-explained manufacturing method. In the following, details of the experiments will be explained that were performed in order to obtain the results depicted in Figures 1 to 5B.

**Gel permeation chromatography (GPC).** GPC measurements were performed using an Agilent 1100 solvent delivery system with a manual injector, isopump, and Agilent 1100 differential refractometer. The Brookhaven BIMwA7-angle light scattering detector was coupled to a size exclusion chromatography (SEC) to measure the molecular weight for each fraction of the polymer that was eluted from the SEC columns. For separation of the polymer samples, three 30 cm columns were used (10 $\mu$m PSS Suprema columns with pore sizes of 100 Å, 1000 Å, 3000 Å). Water was used as mobile phase; the flow rate was set at 1.0 mL/min. All columns were held at room temperature. For each measurement, 100 $\mu$L of samples with concentration of 5 mg mL$^{-1}$ solution was injected. For acquisition of the data from seven scattering angles (detectors) and differential refractometer WinGPC Unity from PSS was used. Molecular-weight distributions were determined by comparison with Pullulan standards (10 different sizes from 342 to 710,000 g mol$^{-1}$). Water was used as a solvent with 0.1 M $NaNO_3$.

**Dynamic light scattering (DLS).** The size of hPP nanoparticles in aqueous solution was measured using a Zetasizer Nano ZS analyzer with an integrated 4 mW He-Ne laser at a wavelength of 633 nm with a backscattering detector angle of 173° (Malvern Instruments Ltd, UK) at 25 °C. For DLS experiments, an aqueous solution of polymer with different concentrations was prepared in Milli-Q water and vigorously stirred for 18 hours at room temperature (25 °C). Solutions were filtered via 0.45 $\mu$m polytetrafluoroethylene (PTFE) filters and used for dynamic light scattering measurements. Disposable UV-transparent cuvettes (Sarstedt AG & Co., Germany) were used for all the experiments.

**Thermogravimetric analysis (TGA).** TGA experiments were performed on a LINSEIS STA PT1600 (TG - DTA/DSC) machine in air atmosphere. The heating rate was set to 10 °C/min. Calibration curves were measured for each sample. Measurements were performed in $Al_2O_3$ crucibles. Mass of samples varied from 15 mg to 20 mg.

**Fourier transform infrared spectroscopy (FTIR).** IR spectra were recorded with Nicolet AVATAR 320 FT-IR 5 SXC (Thermo Fisher Scientific, Waltham, MA, USA) with a DTGS detector from 4000 to 650 cm$^{-1}$. Sample measurement was performed by dropping a solution of compound and letting the solvent evaporate for a few seconds.

**Nuclear magnetic resonance (NMR).** NMR spectra were recorded on a Jeol ECX 400 or a Jeol Eclipse 700 MHz spectrometer. Proton and carbon NMR were recorded in ppm and were referenced to the indicated solvents.

**Confocal laser scanning microscopy (CLSM).** CLSM images were recorded by Leica TCS SP8 with 63x oil-immersion objective lens and analyzed by Leica Confocal Software.

**Microplate reader.** The adsorption of CCK8 assay was measured on a TECAN Infinite M200 Pro microplate reader at the wavelength of 450 nm.

**General procedure for synthesis and purification of hyperbranched polymer.** A catalytic amount of tin octoate ([monomers including Glycidol and $\epsilon$-caprolactone]/[catalyst] : 200/1) solution in toluene was added to a round-bottom flask connected to argon and a vacuum inlet and toluene was evaporated under reduced pressure. Glycidol and $\epsilon$-caprolactone with predetermined molar ratios were then injected to the flask under argon atmosphere and stirred at 50 °C, 90 °C and 120 °C. For example in one of the reactions, 378 mg catalyst in a toluene solution was transferred to a 100 mL round bottom flask. After removing the toluene 5.0 mL (0.15 mol) of glycidol and 4.15 mL (0.037 mol) of $\epsilon$-caprolactone were added to reaction flask. The mixture was stirred (250 rpm) using a mechanical stirrer at 50 °C for 48 h. The crude product was dissolved in methanol and dialyzed against a mixture of methanol/dimethyl formamide (DMF) (70:30) vol % for 24 h using dialysis bag (MWCO =10 kDa) to remove the reactants and low molecular weight side products. Dialysis process was continued for other 24 h against distilled water to remove the remained DMF. Then, the final product (4.5 g) was obtained as a highly viscous and colorless compound upon lyophilization.

**Enzymatic degradation of polymers using Novozyme 435.** The degradation of hPP41-50 was studied according to reported procedure (F. Du, S. Hönzke, F. Neumann, J. Keilitz, W. Chen, N. Ma, S. Hedtrich and R. Haag, Journal of Controlled Release, 2016, 242, 42-49.) in presence of Candida Antarctica Lipase B that had been immobilized on acrylic resin, commercially known as Novozym-435. To a phosphate buffer solution (pH7.4) of polymer (5 mg mL$^{-1}$), 200 wt % of Novozyme-435 and 10 $\mu$L n-butanol were added and the samples were incubated at 37 °C using a BioShake XP® (Biometra) and shaken at 1000 rpm for 3 days. The solution was then filtered centrifuged and freeze-dried and the obtained lyophilisate was analyzed via GPC and $^1$H NMR to monitor the degradation.

**Enzymatic degradation of hPP41-50 using skin lysate.** Degradation of hPP41-50 nanocarriers in presence of skin lysate was investigated according to a previously reported procedure (F. M. Bätz, W. Klipper, H. C. Korting, F. Henkler, R. Landsiedel, A. Luch, U. von Fritschen, G. Weindl and M. Schafer-Korting, European Journal of Pharmaceutics and Biopharmaceutics, 2013, 84, 374-385.). Briefly, freshly excised human skin was homogenized and then 0.5 mg ml$^{-1}$ solution of hPP41-50 was incubated with the skin lysate for 12 h at 37 °C. Samples were centrifuged to separate the solid residues and the supernatant was lyophilized and subsequently analyzed by GPC. hPP41-50 nanocarriers have been also incubated with phosphate buffered saline (PBS) as negative control.

**Covalent conjugation of fluorescein isothiocyanate (FITC) to hPP41-50.** hPP41-50 (50 mg, 0.001 mmol) and fluorescein isothiocyanate (10 mg, 0.02 mmol) were dissolved in 20 mL of dry DMF and stirred at 60 °C for 24 h. DMF was evaporated under reduced pressure and the crude product was dissolved in minimum amount of water and the unreacted FITC was separated through the size exclusion chromatography (SEC) with Sephadex G-25 (GE Healthcare) under ambient pressure and temperature. FITC-labeled hPP41-50 was obtained after lyophilization. $^1$H NMR and fluorescence emission spectroscopy proved that FITC is successfully conjugated to hPP41-50.

**Preparation of Nile red loaded nanocarriers.** hPP41-50 nanocarriers were loaded with Nile red using the solvent evaporation method (Y. G. Bachhav, K. Mondon, Y. N. Kalia, R. Gurny and M. Möller, Journal of Controlled Release, 2011, 153, 126-132; M. Lapteva, K. Mondon, M. Möller, R. Gurny and Y. N. Kalia, Molecular Pharmaceutics, 2014, 11, 2989-3001.). Briefly, an excess of Nile red (50 wt % of polymer) was dissolved in methanol and added dropwise to an aqueous solution of polymer (5 mg mL$^{-1}$) under vigorous stirring. Methanol was then slowly removed by using a rotary evaporator. After equilibration overnight, the solution was centrifuged at 5000 rpm for 15 min and the supernatant was carefully collected, lyophilized and dissolved in methanol for determination the concentration of loaded Nile red by calculated by UV-vis spectroscopy and using calibration curve. Loading capacity was calculated as Nile red concentration divided by polymer concentration.

**Cell viability assays.** All bio-experiments were performed according to the German genetic engineering law and German biosafety guidelines in laboratory, level 1. Dulbecco's Modified Eagle Medium (DMEM), Gibco and fetal bovine serum (FBS) were employed in the following experiments. HaCat cells were cultured in DMEM with 10 % (v/v) FBS and 1 % (v/v) antibiotics. And cells were incubated in a humidified atmosphere with 5 % $CO_2$ at 37 °C. HaCat cells ($1\times10^4$ cells/well) were seeded in a 96-well plate and incubated for 24 h before the tests. hPP41-50 in the culture medium (concentration from 0.1 $\mu$g mL$^{-1}$ to 1000 $\mu$g mL$^{-1}$) were added to the medium-removed 96-well plate and incubated for another 24 h. After that, medium solutions were removed and the cells were rinsed with PBS twice. Subsequently, 100 $\mu$L DMEM with 10 $\mu$L CCK8 solution was added to each well. After incubation for the following 2 hours, the absorption of each well was measured at wavelength of 450 nm with microplate reader. Cells without any treatments and cells treated with DOX-HCl (2 $\mu$g mL$^{-1}$) were used as negative and positive controls. Each sample was measured 3 times.

**Cellular uptake.** HaCat cells were seeded on 8-well plates ($3 \times 10^4$ cells/well). After 24 h, FITC labelled hPP41-50 dissolved in DMEM (10 $\mu$g mL$^{-1}$) was added to the cells. The cells were incubated at 37 °C for different time intervals. Afterwards, the culture medium was removed and the cells were rinsed with PBS. The nucleus of HaCat cells were stained with Hoechst for 30 min and then the cells were observed with CLSM (Leica TCS SP8). Hoechst was excited at 350 nm with the emission at 460 nm and FITC was excited at 490 nm with the emission at 520 nm.

**Skin penetration.** Human skin (obtained from cosmetic surgeries with informed consent, ethics vote EA1/081/13) was punched to discs of 2 cm diameter and mounted onto static-type Franz cells (diameter 15 mm, volume 12 mL, PermeGear Inc., Bethlehem, PA, USA) and experiments was performed according to finite dose approach. In order to investigate the potential dermal drug delivery and skin distribution of hPP41-50, Nile red and FITC were physically encapsulated and covalently attached to the hPP41-50, respectively. The experiments have been performed according to a reported procedure (F. Zabihi, S. Wieczorek, M. Dimde, S. Hedtrich, H. G. Börner and R. Haag, Journal of Controlled Release, 2016, 242, 35-41.) using excised human skin and a conventional base cream served as a reference. All formulations contained the same Nile red concentration (0.001 wt %). Prior to the experiment, human skin was thawed and discs of 2 cm diameter were punched and mounted onto static-type Franz cells (diameter 15 mm, volume 12 mL, PermeGear Inc., Bethlehem, PA, USA) with the horny layer facing the air and the dermis having contact with the receptor fluid phosphate buffered saline pH 7.4 (PBS, 33.5 °C, skin surface temperature about 32 °C) stirred at 500 rpm. After 30 min, 36 µL of the test formulations and reference cream was applied onto the skin surface (finite-dose approach) and remained there for 6 h. Subsequently, the skin was removed from the Franz cells, and the skin surface was gently cleaned. Afterwards, treated skin areas were punched, embedded in tissue freezing medium (Jung, Nussloch, Germany) and stored at a temperature of -80 °C. To determine the polymer penetration, the skin discs were cut into vertical slices of 10 µm thickness using a freeze microtome (Frigocut 2800 N, Leica, Bensheim, Germany). The slices were subjected to normal light and fluorescence light. Nile red and FITC distribution were visualized by fluorescence microscopy.

Figure 1 shows a reaction scheme according to which different hyperbranched polyester polyols (hPPs) were synthesized by cationic ring-opening polymerization of glycidol in the presence of ε-caprolactone and Sn(Oct)$_2$ as catalyst in a one-step reaction. A series of polymerizations using different [glycidol]/[ε-caprolactone] ([G]/[C]) ratios were performed to investigate the role of the reactant molar ratio and the reaction temperature on the polymer structure.

Polymers synthesized by 2/1 and 4/1 molar ratios of [G]/[C] at 50 °C, are nominated as hPP21-50 and hPP41-50, respectively. The polymerizations for ([G]/[C]) 2/1 have been also performed at 90 °C and 120 °C and they are termed as hPP21-90 and hPP21-120, respectively (see Table 1 below). Since the hPP41-50 has been synthesized under milder conditions and showed high water solubility and relatively narrow polydispersity, it was selected for further characterization and skin penetration studies.

IR spectroscopy and different NMR techniques including $^1$H NMR, $^{13}$C NMR, DEPT NMR, DOSY, and $^{13}$C, $^1$H-HSQC spectra clearly showed the incorporation of caprolactone segments into the copolymer. The IR spectrum of hPPs shows absorbance bands at 1100 cm$^{-1}$, 1727 cm$^{-1}$, 2900 cm$^{-1}$, and 3400 cm$^{-1}$, which are assigned to the C-O, carbonyl groups, aliphatic C-H, and end hydroxyl groups, respectively (Figure 2A). Thereby, areas highlighted in light grey are attributed to chemical groups of glycerol residues, and areas highlighted in dark grey are attributed to chemical groups of caprolactone residues.

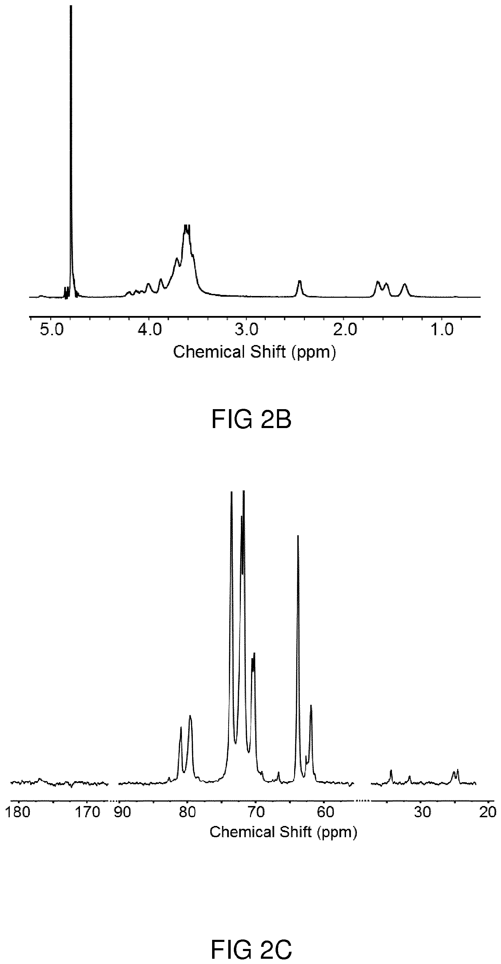

In the $^1$H NMR spectrum of Figure 2B, signals at 1.4 ppm, 1.6 ppm, and 2.5 ppm (highlighted in dark grey) are assigned to methylene protons of caprolactone segments. Signals at 3.4-4.2 ppm and 4.8 ppm (highlighted in light grey) are attributed to the protons of glycerol residues and hydroxyl groups, respectively. Protons of -OCH2 groups in caprolactone segments, which appeared at 4.0 ppm, are covered by glycerol signals. In contrast to homopolymers and block copolymers, signals of caprolactone segments of the hyperbranched polyester polyol are broadened in $^1$H NMR spectra. Additionally, signals of methylene groups in the vicinity of central carbon of caprolactone units are split into two signals (Figure 2B).

In order to make sure that the formation of self-assemblies in water does not affect the $^1$HNMR spectra, this measurement has been also performed in deuterated DMSO and DMF. It could be seen that the spectra are similar to that of measured in D$_2$O (data not shown).

In the $^{13}$C NMR spectrum of Figure 2C, signals at 22-35 ppm and 177 ppm are assigned to caprolactone segments (highlighted in dark grey). In this spectrum, the glycerol signals appeared at 60-82 ppm (highlighted in light grey).

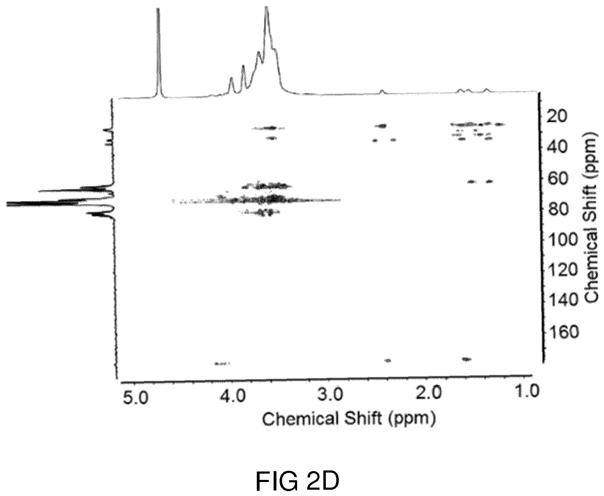

Two-dimensional NMR techniques, including heteronuclear single quantum coherence spectroscopy (HSQC) and diffusion-ordered NMR spectroscopy (DOSY), were used to study the correlation of caprolactone and glycerol segments in hPP41-50. The cross-relaxations observed in HSQC NMR spectra confirmed the correlation and covalently bonding of caprolactone and glycidol in the polymer backbone (Figure 2D). A correlation of -CH protons of glycidol units with the caprolactone carbons indicated a similar diffusion coefficient of signals of caprolactone and glycerol segments measured by DOSY NMR. This is another proof of a covalent attachment of these monomers in the polymer architecture.

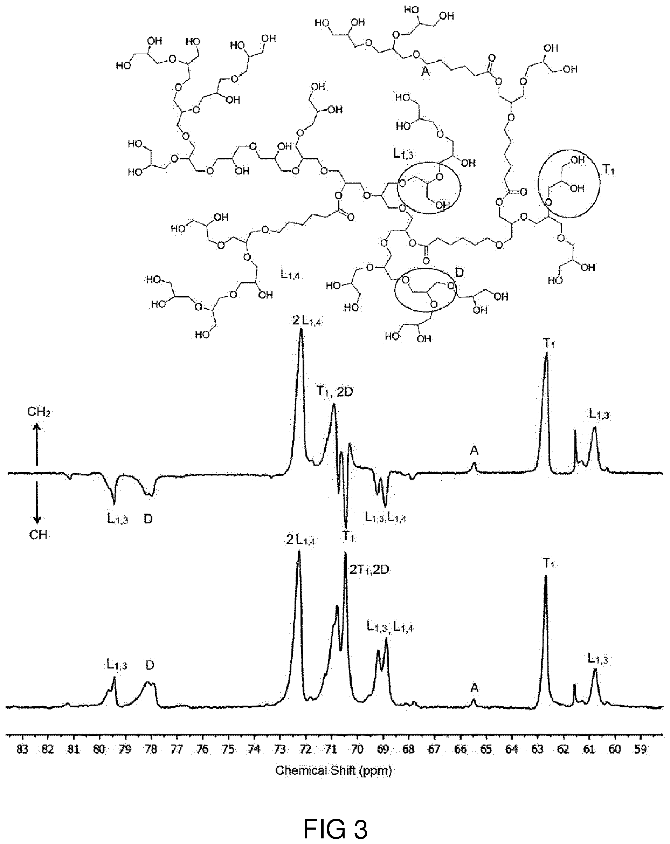

Polymerization of glycidol would result in a branched architecture. Since the branched structure of the synthesized hPP originated from glycidol monomer, the degree of branching (DB) can be calculated by using the same methods that are generally used for polyglycerol. Therefore, in order to distinguish between methylene and methine carbon atoms and to determine the different structural units such as linear (L), dendritic (D), and terminal (T) units, as well as a degree of branching of polymer, inverse-gated (quantitative) $^{13}$C NMR together with DEPT $^{13}$C NMR have been used and the degree of branching was calculated using the method reported by Sunder et al. (A. Sunder, R. Hanselmann, H. Frey and R. Mülhaupt, Macromolecules, 1999, 32, 4240-4246.). The results are depicted in Figure 3.

As shown in Table 1, the feed molar ratio does not show any effect on the polymer composition and structure. However, increasing the reaction temperature changes the caprolactone content of the polymer and the relative abundance of different structural units. This could be due to different reactivity of glycidol and caprolactone. The DBs of hPPs are in the range of 0.43-0.53, which indicates the formation of hyperbranched polymers. Due to the AB$_2$ structure of glycidol, copolymers with higher glycidol content possess a higher DB (Table 1).

**Table 1.** Relative abundance of terminal, linear and dendritic units and effect of reaction temperature and glycidol/ε-caprolactone molar ratios on the hPPs composition.

| Structural units | Shift (ppm) | hPP 21-50 | hPP 41-50 | hPP 21-90 | hPP 21-120 |
|---|---|---|---|---|---|
| L$_{1,3}$ | 79-80 | 1.00 | 1.00 | 1.00 | 1.00 |
| D | 77.5-79 | 1.85 | 2.18 | 1.59 | 1.89 |

(continued)

| Structural units | Shift (ppm) | hPP 21-50 | hPP 41-50 | hPP 21-90 | hPP 21-120 |
|---|---|---|---|---|---|
| $2L_{1,4}$ | 72-73.5 | 4.97 | 5.07 | 5.16 | 6.31 |
| $2D, 2T_1$ | 70-71.5 | 6.71 | 8.61 | 7.04 | 8.83 |
| $L_{1,3}, L_{1,4}$ | 68.5-70 | 2.19 | 3.55 | 2.43 | 4.00 |
| $T_1$ | 62-63 | 1.80 | 2.38 | 2.21 | 2.62 |
| $L_{1,3}$ | 60-61 | 0.73 | 1.20 | 0.91 | 1.71 |
| Degree of branching [a] | | 0.53 | 0.54 | 0.47 | 0.43 |
| Terminal units (%) | | 21 | 26 | 26 | 26 |
| Dendritic units (%) | | 29 | 27 | 23 | 23 |
| Linear 1,3 units (%) | | 11 | 15 | 13 | 13 |
| Linear 1,4 units (%) | | 39 | 32 | 38 | 38 |
| Feed molar ratio [Gly]/[CL] | | 2/1 | 4/1 | 2/1 | 2/1 |
| Reaction temperature (°C) | | 50 | 50 | 90 | 120 |
| Caprolactone content (%) [b] | | 5 | 6 | 9 | 16 |
| Yield (%) | | 43 | 45 | 50 | 55 |
| Mn (kDa) (GPC) | | 20 | 20 | 64 | 66 |
| PDI [c] (GPC) | | 1.7 | 1.4 | 2.4 | 2.5 |

[a] Degree of branching (DB) was calculated from an inverse-gated $^{13}$C NMR analysis.

[b] Caprolactone content was calculated from $^1$H NMR integrals.

[c] Determined from GPC in water solution calibrated with pullulan molecular weight standards. PDI = Mw/Mn.

According to $^1$H NMR spectroscopy, the caprolactone content of hPPs did not exceed 16 %, even at low glycidol/caprolactone molar ratios and high temperatures (Table 1). This is due to the higher reactivity of glycidol, as a highly strained monomer, in the ring-opening polymerization.

Thermogravimetric analysis (TGA) showed similar thermal behavior for all samples. Although the decomposition temperatures of polycaprolactone and polyglycerol are different, there was only one main weight-loss temperature for the synthesized hPPs. This is a clear indication for the random incorporation of caprolactone segments in the backbone of the copolymer rather than the formation of a block copolymer. Otherwise the produced polymer would have decomposed at two different temperatures and would have shown two weight losses.

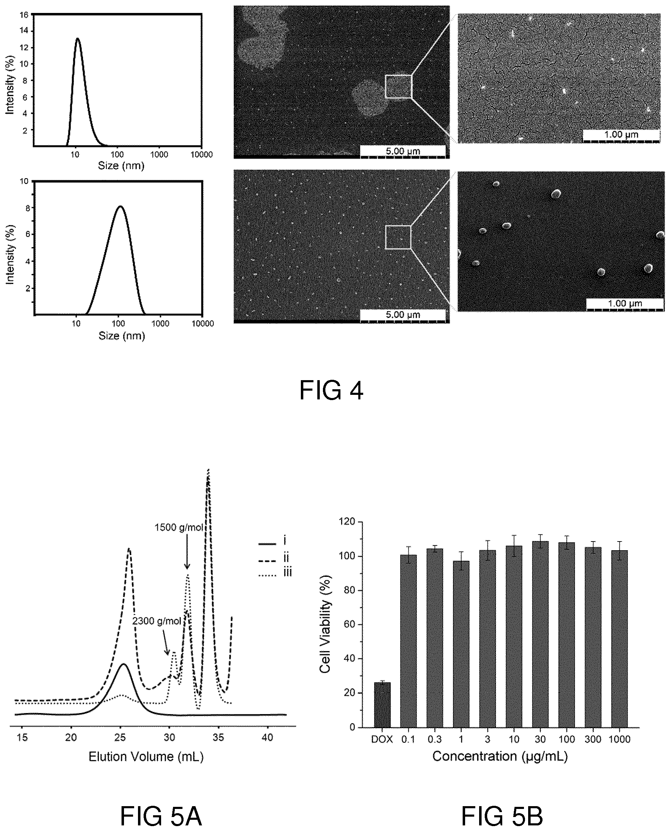

The particle size of the synthesized polymers as well as their aggregation in aqueous solution were studied using dynamic light scattering (DLS) and scanning electron microscopy (SEM). DLS of hPPs, synthesized by different monomer feed ratios at 50 °C, was almost the same. However, the size of aggregates in aqueous solution increased upon raising the reaction temperature, which also increased the caprolactone content of the polymers. In consistence with the DLS results, SEM images showed that hPPs assembled in the form of spherical particles in the range of 20-100 nm (cf. Figure 4).

**In vitro biodegradability.** Biodegradability of polymers and nanomaterials is an important parameter, which should be taken into account for their biomedical applications. The degradation test is helpful, not only to prove the biodegradability of polymers but also to infer the location of ester bonds in the polymer structure. Therefore, degradation of polymers was investigated under acidic and enzymatic conditions, and the degradation products were analyzed by GPC and NMR. While the synthesized polymers were stable in acidic conditions (pH = 5.4) for one week, the results of GPC, NMR and skin penetration tests showed that they break down to smaller segments in the presence of Candida Antarctica Lipase B (Novozyme 435) and skin lysate. hPP41-50 was incubated with Novozyme 435, and then a $^1$H NMR spectrum of degradation products was recorded. The appearance of a set of new signals in $^1$H NMR proved an ester bond cleavage and carboxylic acid formation due to polymer degradation. The degree of degradation was calculated using peak area ratio of signals of protons in the alpha position to the carbonyl groups of ester (2.44 ppm) and carboxylic acid (2.15 ppm). Correspondingly, the degree of degradation for hPP41-50 could be calculated to be 33 % after one week from the following equation:

$$\text{Degree of degradation} = \frac{\int 2.15\,ppm}{\int 2.15\,ppm + \int 2.44\,ppm}$$

Measuring the molecular weight of the degradation products was further proof of the degradation of polymers. hPP41-50 degraded to segments that had molecular weights much lower than the pristine polymer (Figure 5A). Regardless of the enzyme type, GPC diagrams showed a similar breaking pattern for hPP41-50. These results proved that cleaving the ester bonds caused biodegradability of the polymers and that there were also caprolactone segments in the backbone of hPPs.

**Biocompatibility of hPP.** As pure polyglycerols and pure polycaprolactone are well known biocompatible materials, the synthesized hPPs were expected to be non-toxic as well. To obtain information about the biocompatibility of the synthesized polymers, CCK8 assays with HaCaT cells (keratinocytes from adult human skin) were carried out. As can be seen in Figure 5B, no significant cytotoxicity for hPP41-50 was observed up to a concentration of 1000 $\mu$g $\cdot$ mL$^{-1}$ after an incubation time of 24 hours. Cells without any treatment were considered as a negative control and cells incubated with DOX-HCl (5 $\mu$g mL$^{-1}$) was employed as a positive control. This shows the biocompatibility for hPPs and their great potential for further biomedical applications.

**Cellular uptake of hPP41-50.** Cellular uptake and intercellular distribution of polymers incubated with HaCaT cells were investigated using confocal laser scanning microscopy (CLSM). Fluorescein isothiocyanate was conjugated to polymers, and then HaCaT cells were incubated with hPP41-50-FITC for different time intervals. Polymers were efficiently and quickly taken up by HaCaT keratinocytes cells, they were mostly accumulated in the cytoplasm and perinuclear space. In order to study the transfer ability of polymers, they were non-covalently loaded with Nile red, and the cellular uptake of encapsulated dye was evaluated by CLSM. The resulting data showed that Nile red was efficiently transferred to the cells upon different incubation times.

These results show the high potential of the synthesized polymers for loading of hydrophobic molecules, due to the caprolactone segments in their backbone, and their ability to transfer the loaded agents into the cells. Because of these advantages and their biodegradability as well as their biocompatibility, the synthesized polymers are of great interest for the biomedical applications. Therefore, their application as dermal delivery systems was investigated.

**Skin penetration.** In order to study the skin penetration of the synthesized polymers, as well as the effect of enzymatic activity of stratum corneum on the penetration and cargo release, hPP41-50 was either covalently labelled with FITC or loaded with Nile red. Then penetration of polymer into the frozen skin (with reduced enzymatic activity) and fresh skin was visualized using fluorescence microscopy. The fluorescence microscopy images of hPP41-50-FITC conjugate showed that hPP41-50 nanocarriers penetrated into the stratum corneum of frozen skin after 6 hours. Meanwhile, the red fluorescent signal of the Nile red-loaded hPP41-50 could be detected in the viable epidermis (VE)). As a result, hPP41-50 remained in stratum corneum but it enhanced the transport of Nile red into the VE layer. This means that the polymers did not penetrate the viable layers of skin. Therefore they do not cause adverse effects on those layers. Additionally, the higher penetration of Nile red into the deeper layers of fresh skin, which is enzymatically more active compared to frozen skin, are considered to be the effect of the enzymatic activity of stratum corneum on the degradation of the polymers or generally faster transport mechanisms in fresh skin.

**Conclusions.** Biodegradable hyperbranched polyester polyols were synthesized through one-pot, ring-opening polymerization of glycidol in the presence of different amounts of $\varepsilon$-caprolactone. The biocompatibility tests showed that the synthesized polymers were biocompatible and suitable for biomedical applications. Due to their amphiphilicity, the synthesized polymers were able to load hydrophobic molecules, i.e. to act as nanocarriers, and to improve the penetration of these molecules into skin. The skin penetration tests showed that the novel nanocarriers remained in the stratum corneum, while they enhanced penetration of loaded molecules to deeper skin layers. This property makes hPPs an appropriate intradermal delivery system without adverse effect on viable skin layers.

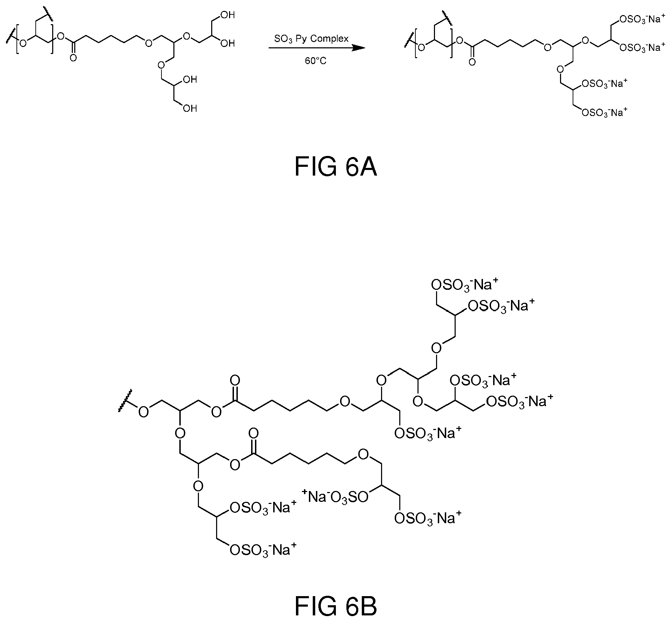

Figures 6 to 15 will be explained in connection to an exemplary embodiment relating to different sulfated hyperbranched polyester polyols, i.e., final products according to an aspect of the present invention resulting from the second step of the above-explained manufacturing method. In the following, details of the experiments will be explained that were performed in order to obtain the results depicted in Figures 6 to 15.

**General procedures.** $^1$H NMR spectra were recorded on a Bruker AMX 500 (Bruker Corporation, Billerica, MA, USA), Jeol ECP 500 (JEOL (Germany) GmbH, Freising, Germany) or a Bruker Avance III 700 (Bruker Corporation, Billerica, MA, USA). Chemical shifts $\delta$ were reported in ppm using the deuterated solvent peak as the internal standard. Elemental analysis was performed on a VARIO EL III instrument (Elementar, Hanau, Germany). DLS and $\zeta$-potential measurements were carried out on a Zetasizer Nano ZS (Malvern Instruments Ltd., Worcestershire, U.K.) equipped with a He-Ne laser (633 nm) using backscattering mode (detector angle 173°).Particle size was measured in UV-transparent disposable cuvettes (Plastibrand®micro cuvette, Brand GmbH + Co KG,Wertheim, Germany) at 25 °C. Samples were dissolved in phosphate buffer (PB, 10 $\times$ 10 -3 M, pH 7.4) at a concentration of 1 mg/mL. The solutions were filtered once a 0.2 $\mu$m RC syringe filter. Samples were equilibrated for 120 s at 25 °C; subsequently, the measurement was performed with 13 scans per sample. For $\zeta$-potential measurements samples were dissolved in phosphate buffer (PB, $10 \times 10^{-3}$ M, pH 7.4) at a concentration of 1 mg/mL. The solutions were measured by applying an electric field across the polymer at 25 °C in folded DTS 1060 capillary cells (Malvern Instruments Ltd., Worcestershire, U.K.). Data evaluation was performed with Malvern Zetasizer Software 6.12. The stated values and standard deviations are the mean of three independent measurements with 15 scans each and are based on the Smoluchowski model.

**Synthesis of sulfated hPPS.** hPP was prepared as explained with respect to Figures 1 to 5B. For sulfation, the polymer was pre-treated overnight in vacuum, at 60 °C. Dry DMF was added and the solution was homogenized at 60 °C for 1hour. A solution of $SO_3$ pyridine complex (1.5 eq/OH groups) in dry DMF was added over a period of 3 hours. The reaction was stirred at 60 °C for 24 hours, under argon atmosphere. The reaction was quenched with water and the pH was adjusted to 7 by addition of $NaOH_{aq}$.
The solvent was evaporated under reduced pressure and the product was dissolved in brine. Dialysis was performed against NaCl-solution (MWCO = 2kDa), using an ever decreasing NaCl concentration, until medium was changed with distilled water. The product was lyophilized and obtained as a yellow-brown solid. Based on the sulfur content, the degree of sulfation was determined by elemental analysis.

**hPP Functionalization, Dye Conjugation and Sulfation.** The dye conjugate was prepared as follows. hPP (0.1g, 0.012 mol OH, 1 eq.) was dissolved in dry DMF (2 mL), then triethylamine (0.07 mL, 2 eq./20 %OH groups) was added and the solution cooled in an icebath. Mesyl chloride (0.02 mL, 1 eq.) was added and the reaction was magnetically stirred over night at room temperature, under an argon atmosphere. The product was dialyzed for 24 h against MeOH (MWCO = 3.5-5 kDa).

The mesyl-derivate was dissolved in DMF (5 mL) and sodium azide (0.08g, 0.0012 mol, 5 eq.) was added. The reaction was performed at room temperature for 24h. After filtration, the product was dialyzed for 2 days, first in $H_2O$, then in MeOH (MWCO = 3.5-5 kDa).

The azide-derivate was dissolved in water/THF mixture and PPh3 (0.2g, 0.00076 mol, 5 eq.) was added. The solution was stirred for 48h, and then dialyzed against MeOH (MWCO = 3.5-5 kDa). The degree of functionalization (DF) was determined to be 6 %.

Amine-factionalized hPP (8.5 mg) was dissolved in DMF (1 mL).The ICC dye was dissolved in a mixture of $H_2O$/DMF (2 mg, 2 eq.) and together with DIPEA (2 eq.) added to the reaction mixture. The solution was stirred overnight at room temperature. The product was purified by dialysis in water (MWCO = 2 kDa). The conjugation was controlled by reversed-phase thin layer chromatography (RP TLC). Afterwards, the product was sulfated using the procedure described.

**Cell culture, cellular uptake and fluorescence-activated cell sorting.** Viability tests were performed using epithelial human lung cancer cell line A549 and epithelial human colorectal adenocarcinoma cell line Caco-2. Propagation was performed for A549 cells in Dulbecco's Modified Eagle Medium (DMEM, low Glucose) with 10 % fetal bovine serum (FBS) and 1 % penicillin/streptomycin. Cells were seeded in medium at $1 \times 10^5$ cells mL$^{-1}$, cultured at 37 °C with 5 % $CO_2$ and split 1:5 two times a week. Propagation of Caco-2 cells was performed in Minimum Essential Medium (MEM) with 1 % MEM Non-essential Amino Acid Solution 100x, 1 % Sodium Pyruvate, 20 % fetal bovine serum (FBS) and 1 % penicillin/streptomycin. Cells were seeded in medium at $1 \times 10^5$ cells mL$^{-1}$, cultured at 37 °C with 5 % $CO_2$ and split 1:5 two times a week. Viability was determined using CCK-8 kit. Cells were cultured in 96-well plates at a density of $4 \times 10^4$ cells/mL (A549) or $1 \times 10^5$ cells/ mL (Caco-2) with normal culture medium or medium containing the sulfated polymer for 24h. Absorbance measurements at wavelength of 450 nm and a reference wavelength of 650 nm enable viability quantification. Tests were conducted using a Tecan plate reader (Infinite pro200, TECAN-reader Tecan Group Ltd., Männedorf, Switzerland). Measurements were performed in pentaplicate and repeated thrice. The cell viability was calculated by setting the negative control, corresponding to cells cultivated in their own medium, equal to 100 %; the positive control is represented by cells treated with 0.1 % SDS. The background signal was subtracted prior to calculation.

Cellular uptake was verified using a confocal laser scanning microscope (Leica SP8). Cells were seeded in 24-well plates at $1 \times 10^4$ cells /500 µL, on a transparent dish and incubated with culture medium or medium containing dye-labelled substance (final concentration 0.5 mg/mL) for 1, 4 and 24 h at 37 °C. The culture medium was then replaced with 500 µL staining agent solution, followed by several washes with PBS after ten minutes. 250 µL of a 4 % PFA (p-Formaldehyde) solution was added for crosslinking, then, after 15 minutes, the solution was replaced with 4',6-diamidino-2-phenylindole (DAPI) and incubated for 20 minutes. After several washes, the dishes were glued on microscope glass slide and used for analysis. Images of different groups were acquired with the same laser and detector settings using the Leica LAS X software.

For flow cytometry analysis, cells were seeded at a density of $5 \times 10^4$ cells / mL (A549) or $1 \times 10^5$ cells / mL (Caco-2) and cultivated for 24 h at 37 °C. After washing with PBS, cells were detached with trypsin, recovered with PBS and centrifuged for 4 minutes at -4 °C, at 140 rpm. After supernatant removal, cells were suspended in PBS and immediately analyzed in a Accuri C6 analysis instrument.

**Competitive Selectin Ligand Binding Assay.** The measurements of this assay were estimated by surface plasmon resonance (SPR) by using a BIAcore X100 (GE Healthcare Europe GmbH, Freiburg, Germany) at 25 °C and a constant flow rate of 20 $\mu$l/min. Since it has been described in detail by S. Enders *et al.* (S. Enders, G. Bernhard, A. Zakrzewicz, R. Tauber, Biochim. Biophys. Acta, Gen. Subj. 2007, 1770, 1441.), it will be described shortly: to mimic leukocytes, Au nanoparticles (15 nm diameter, Aurion Immuno Gold Reagents & Accessories, Wageningen, The Netherlands) were coated with L-selectin-IgG chimera (R&D Systems GmbH, Wiesbaden, Germany) whereas the SPR sensor chip's surface imitates the vascular endothelium. Therefore, SiaLe$^x$-(20 mol-%)-PAA-sTyr-(5 mol-%)) (PAA = polyacrylamide) (Lectinity Holdings, Inc., Moscow, Russia) on the measuring channel and *N*-acetyllactosamine-PAA (Lectinity Holdings) on the reference channel were immobilized. The preparation of the selectin nanoparticles and the measurements were conducted with 20 mM HEPES-Buffer (pH 7.4) containing 150 mM NaCl and 1 mM CaCl$_2$. To assess the inhibitory potential of polycaprolactones against the binding of L-selectin to its ligand the substances were preincubated. The results of L-selectin binding without inhibitor were given in response units and set to 100 %, after reference subtraction. Due to the inhibitor concentrations (0.1 pM-100 nM) the reduced binding signal was calculated as x % binding of the control. Hence, the necessary inhibitor concentration for binding reduction of 50 % was set as IC$_{50}$ value. Measurements were done in duplicates.

**Blood Clotting Assay.** The influence of different polycaprolactone derivatives on blood coagulation was estimated by determining the partial thromboplastin time (PTT). Therefore an Amelung coagulometer (Type 410A4MD, Lemgo, Germany) was used. A mixture of 100 $\mu$l standard citrated human plasma, 100 $\mu$L Actin FS solution (both from Siemens Healthcare, Erlangen, Germany) and optionally 4 $\mu$l of the polycaprolactone was incubated (3 min, 37 °C). The final concentration of the test compounds was ranging from 0.05 $\mu$g/ml to 50 $\mu$g/ml. The anticoagulant heparin (Sigma-Aldrich, St. Louis, USA) was used as a control in the same concentration range. By adding 100 $\mu$l pre-warmed (37 °C) CaCl$_2$ solution the blood coagulation was started. Measurements for each concentration were determined in triplicates. Finally the untreated standard human plasma conduced to control and its clotting time was set to 100 %.

**Complement Activity.** The Complement Activity was tested by using the WIESLAB® Complement system classical pathway kit is described in the manufacturer's instructions in detail, briefly: human serum was diluted 1:101 with Diluent CP and treated, if applicable, with different final concentrations (1000, 500, 250, 100, 50 nM) 1:50 of the polycaprolactones. After 5 min pre-incubation step at room temperature, they were distributed into respective manufacturer's well-plate and were again incubated (1 h, 37 °C). Subsequent, the wells were washed three times with the washing buffer and refilled with 100 $\mu$l conjugate solution. Another incubation step (30 min, 20-25 °C) was followed by a second washing step as described before. Finally 100 $\mu$l of the substrate solution were added and incubated for 30 minutes at room temperature before the well absorbance at 405 nm was measured on a microplate reader (Tecan). The untreated serum was set to 100 % activity and the potency of the inhibitor concentrations was calculated. The concentration where the activity was reduced by 50 % was set to the IC$_{50}$ value.

**Degradation Studies.** The enzymatic degradation studies were performed in PB solution at pH = 7.4, at 37 °C. Probes (C =5 mg/mL) were incubated wit 50$\mu$L Human Leukocyte Esterase (HLE) solution. For each time point, a probe was first shock-frozen in liquid nitrogen and then lyophilized and the degradation was analyzed by $^1$H NMR.

**Synthesis and characterization of sulfated hPPs.** Due to its favorable characteristics such as the biocompatibility, biodegradability and the possibility to load a host cargo, the hyperbranched polyester polyol with a glycidol to caprolactone ratio of 4/1, synthesized at 50 °C, was chosen as candidate for further functionalization studies. The conversion of the hydroxyl groups to sulfates was performed by the aid of a SO$_3$ pyridine complex. The one-pot process is schematically depicted in Figure 6A. Figure 6B shows a bigger detail of the chemical structure of an exemplary embodiment of a hyperbranched polyester polyol derivative. The amount and the location of caprolactone residues is only to be understood exemplarily.

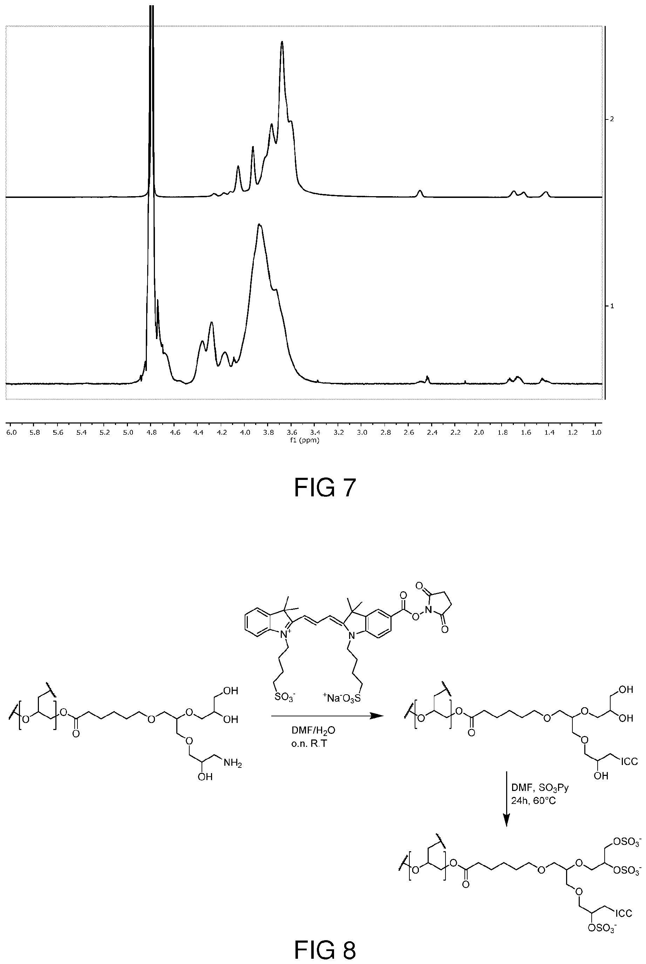

The successful transformation of the hydroxyl groups was confirmed by $^1$H NMR analysis; it was also possible to see that the process did not lead to degradation of the overall polymer structure. In Figure 7 the $^1$H NMR spectra before (upper spectrum) and after (lower spectrum) the sulfation are depicted. A shift to higher chemical shifts assigned to the glycerol residues of the backbone, in the region between 3 and 4.5 ppm, as well as changes in the signals at 4.2 to 4.4 ppm can be observed, which corresponds to the formation of the sulfate groups.

In order to further confirm the successful sulfation process, elemental analysis was performed to determine the sulfur content of the polymer. Based on that data, it was possible to define the degree of sulfation (DS) of each polymer. Finally, GPC measurement enabled the determination of the molecular weight and polydispersity of the polymers.

Table 2 summarizes the physical properties of the synthesized compounds described herein.

**Table 2.** Overview on the synthesized products.

| Compound | $M_n$ [kDa] | $M_w$ [kDa] | PDI | Sulfur content [%] | DS |
|---|---|---|---|---|---|
| **High MW hPP** | 47 | 62 | 1.33 | 0 | 0 |
| **hPP$_{50kDa}$S$_{80\%}$** | 57 | 80 | 1.41 | 14.3 | 80 |
| **hPP$_{50kDa}$S$_{95\%}$** | 54 | 70 | 1.29 | 17.6 | 95 |

(continued)

| Compound | $M_n$ [kDa] | $M_w$ [kDa] | PDI | Sulfur content [%] | DS |
|---|---|---|---|---|---|
| Low MW hPP | 13 | 29 | 2.15 | 0 | 0 |
| $hPPP_{30kDa}S_{90\,\%}$ | 34 | 51 | 1.49 | 16.0 | 90 |

Two derivatives were synthesized from a high molecular weight starting material (50 kDa) and one from a low molecular weight starting material (30 kDa). The polymers with high molecular weight differ consistently in their degree of sulfation (80 % or 99 %, respectively); the polymer with low molecular weight possesses an intermediate functionalization (90 %).

The polymers were also investigated in relation to their size and charge in aqueous solution. The study was conducted in phosphate buffer at pH 7.4 (PB).

In Table 3, the size and surface charge for synthesized compound, measured by DLS, is displayed. It clearly shows similar sizes around 10 nm, slightly bigger for the non-sulfated precursors; moreover, all particles display a strong negative charge ranging from -30 to almost -70 mV for the sulfated samples. A possible tendency to aggregate in solution is, however, hypothesized.

**Table 3.** Properties of polymers in solution.

| Compound | Size in PB [nm] | Z-potential in PB [mV] |
|---|---|---|
| **High MW hPP** | 17.04 | -11.49 |
| $hPP_{50kDa}S_{80\,\%}$ | 10.66 | -49.53 |
| $hPP_{50kDa}S_{95\,\%}$ | 13.27 | -33.53 |
| **low MW hPP** | 10.35 | -6.62 |
| $hPP_{30kDa}S_{90\,\%}$ | 8.99 | -67.4 |

**hPPs Functionalization, Dye Conjugation and Sulfation.** A polymer-dye conjugate was also synthesized for performing cellular uptake studies. First of all, a small percentage of hydroxyl groups were converted to amine groups, using a multistep procedure which involves the formation of mesyl groups, their nucleophilic substitution with azide and the subsequent reduction of the latter to amine. A degree of functionalization equal to 5 % was sufficient to enable the conjugation of an ICC NHS ester dye. The synthetic pathway is shown in Figure 8. The elemental analysis of the labeled polymer showed a degree of sulfation of 88 %.

**Biological evaluation.** The biocompatibility of products that shall be used in vivo is a fundamental prerequisite. In order to obtain deep information about this aspect, the polymers were first tested in vitro using different cell lines. In particular, attention was pointed at any possible cytotoxic effect, which is expected to be shown by affecting the viability of cells while cultivated in presence of a possible toxic agent.

The cell viability in the presence of the sulfated polymer was tested using two epithelial cell lines, namely A549 (human lung carcinoma) and Caco-2 (colorectal adenocarcinoma) cells. Medium without additions was used as negative control (NC); a solution of 0.1 % sodium dodecyl sulfate (SDS) was used as positive control (PC).

Cells were placed in 96 wells plate, grown for 24 hours and the polymer solution was then added. The viability was investigated 24 hours after the treatment, using the CCK-8 test: its response is based on the activity of cells in converting a tetrazolium salt to a formazan dye. The test was performed using three different concentrations of polymer solution, ranging from 5 $\mu$g/mL to 500 $\mu$g/mL; each concentration was tested as pentaplicate and the testes were performed three times.

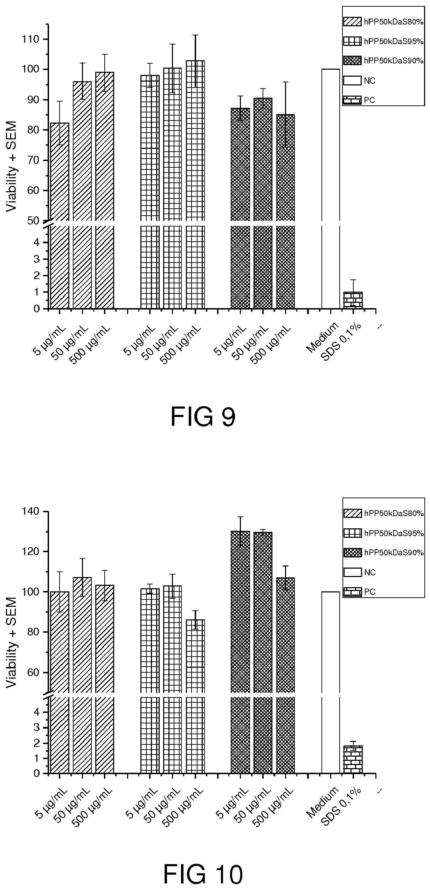

As can be seen from Figure 9, the sulfated polymers showed no or only a very slight toxicity at all concentrations. In some case, the viability resembles to be higher than that exhibited by untreated cells, therefore it is possible to pose that the compound could also stimulate the cellular activity. Similar results could be observed when testing the viability of Caco-2 cells upon incubation with the sulfated polymers, as displayed in Figure 10. Also in this case, the tested sulfated polymers do not to induce cell death. Moreover, it could be observed that the cell viability is not negatively influenced neither by the molecular weight nor the degree of sulfation of the polymer, confirming the biocompatibility of all products. Furthermore, it appears that $hPP_{30kDa}S_{90\,\%}$ has a positive effect on the cell viability.

**Cellular uptake and Flow Cytometry.** Another examined aspect is the possibility for the polymer to be internalized by a cell (cellular uptake). For that aspect, the cells were incubated with a low molecular weight polymer which had been conjugated with a red dye prior to sulfation. The uptake was investigated over 1 hour, 4 hours and 24 hours after incubation. The nucleus of cells was stained in blue and the membrane in green in order to localize the conjugated polymer. The overlapping of the signal of membrane and polymer results in yellow color. Images were taken using a confocal microscope.

Already after one hour a light localization of the polymer in the cell membrane could be observed in case of A549 cells. Moreover, as the incubation time increased, an increased cellular uptake could be observed with its best results after 24 hours.

The Caco-2 cells showed a slightly different behavior. Even though a small uptake could be observed in the first hours, a significant uptake could be observed only after 24 hours. These cells resemble to possess a much lower activity, which could be correlated to their much higher duplication time. Moreover, the cells seem to agglomerate upon polymer contact; uptake can mainly be observed in the peripheral region of agglomerated cells.

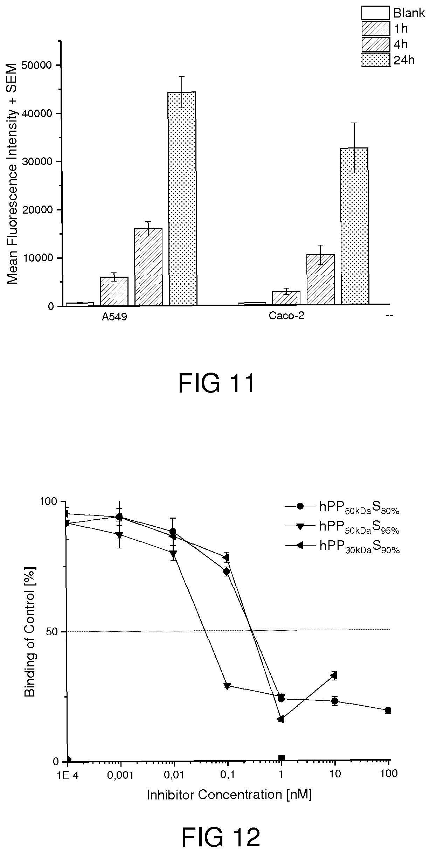

A further investigation was performed using flow cytometry. The cells were incubated for the same timeframe as in the previous experiments. The results are shown in Figure 11. They confirm the previous findings that an increased polymer uptake occurred with increasing time.

**L-selectin Inhibition experiments via SPR Measurements.** The family of selectins plays a key role in the recruitment of leukocyte to the target of inflammation. In order to avoid the undesired activity, it is possible to interfere with this mechanism by blocking the receptor of selectins. In a competitive SPR based assay, it is possible to determine the $IC_{50}$ of a product, which corresponds to the quantity of substance necessary to inhibit the 50 % of the activity of L-selectin.

Different performances in L-selectin inhibition could be observed depending on the degree of sulfation and the molecular weight of the polymer (Figure 12). The best result could be observed for the high molecular weight compound with the highest degree of sulfation (HMW-HS; $hPP_{50kDa}S_{95\%}$); the $IC_{50}$ value was 30 pM. Decreasing the sulfate content or the molecular weight resulted in a diminution of the performance, which led to IC50 values of 0.3 nM for the high molecular weight compound with low degree of sulfation (HMW-LS; $hPP_{50kDa}S_{80\%}$) or the low molecular weight compound (LMW; $hPP_{30kDa}S_{90\%}$). Interestingly, both latter polymers display almost the same performance. It appears that a lower sulfur content can be compensated by a higher molecular weight, and vice versa.

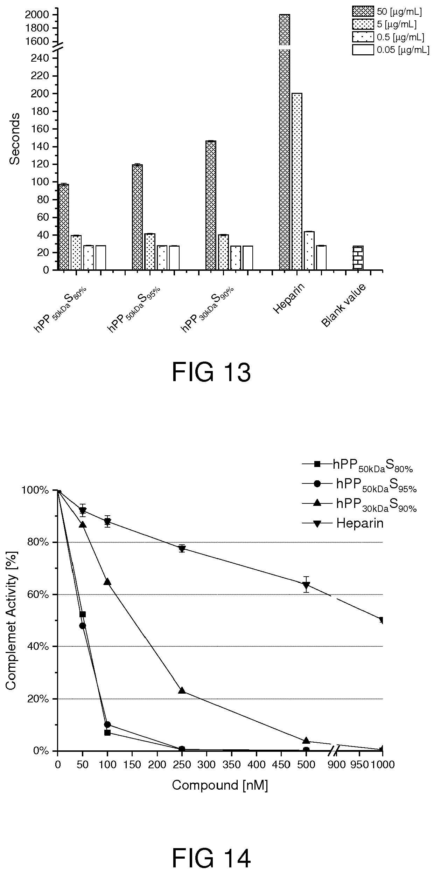

**Blood Clotting Assay and Complement Activity.** Possible effects of the sulfated polymers on blood coagulation and complement activation was tested. As can be seen in Figure 13, all sulfated samples exhibit an influence on blood coagulation time; however, the effect is remarkable only at the highest tested concentration, corresponding to 50 $\mu$g/mL. In comparison, heparin exhibits at the same concentration an effect which is more than 10 times higher; even at a 10 times smaller concentration has heparin still has a stronger effect than the sulfated polymers.

An undesired effect which usually arises during an inflammatory process is the activation of the complement system. Compounds which are able to disturb the complement activation are highly desired. As can be seen in Figure 14, all tested sulfated polymers are able to inhibit complement activation and show a better performance than heparin.

**Degradation studies.** The biodegradability of a compound is a key aspect concerning its utilization in living systems. Therefore, the possibility to degrade the polymers in presence of a natural occurring enzyme was examined. As explained in connection to Figure 5A, it could be demonstrated that the non-sulfated polymers can undergo enzymatic degradation. For confirming that the functionalization had not interfered with this property, the sulfated polymers were incubated with human leukocyte esterase in PBS buffer at pH 7.4

NMR spectra of the sulfated polymers at different times of incubation with the esterase were evaluated. A signal at 2.5 ppm, representing the hydrogen bound to the carbon in alpha position to the ester group, was disappearing over time. Even though after 4 hours a small signal was still recognizable, no such signal could be observed at all after 48 hours. Moreover, at the same time it is possible to observe the appearance of a new tiny signal at 2.2 ppm, which can be assigned to a carboxyl group which is formed upon ester cleavage. Finally, it was also possible to detect a signal in the region between 0.5 and 2 ppm that could be assigned to the esterase. This signal decreased with time, indicating the deactivation of the enzyme and a loss of enzymatic activity.

**List of references cited in the preceding sections or giving otherwise appropriate background information**

1. WO 2012/031245 A1

2. US 2015/0037375 A1

3. US 2016/0331875 A1

4. Z. Li & J. Li, The Journal of Physical Chemistry B, 2013, 117, 14763-14774.

5. D. Wilms, S.-E. Stiriba and H. Frey, Accounts of Chemical Research, 2010, 43, 129-141.

6. I. Gitsov, Journal of Polymer Science Part A: Polymer Chemistry, 2008, 46, 5295-5314.

7. E. Mohammadifar, A. Nemati Kharat and M. Adeli, Journal of Materials Chemistry B, 2015, 3, 3896-3921.

8. N. Kurniasih, J. Keilitz and R. Haag, Chemical Society Reviews, 2015, 44, 4145-4164.

9. S. Stefani, I. N. Kurniasih, S. K. Sharma, C. Bottcher, P. Servin and R. Haag, Polymer Chemistry, 2016, 7, 887-898.

10. R. A. Shenoi, B. F. L. Lai and J. N. Kizhakkedathu, Biomacromolecules, 2012, 13, 3018-3030.

11. R. K. Kainthan, E. B. Muliawan, S. G. Hatzikiriakos and D. E. Brooks, Macromolecules, 2006, 39, 7708-7717.

12. R. K. Kainthan and D. E. Brooks, Biomaterials, 2007, 28, 4779-4787.

13. E. Mohammadifar, A. Bodaghi, A. Dadkhahtehrani, A. Nemati Kharat, M. Adeli and R. Haag, ACS Macro Letters, 2016, DOI: 10.1021/acsmacrolett.6b00804, 35-40.

14. C. Dingels, S. S. Müller, T. Steinbach, C. Tonhauser and H. Frey, Biomacromolecules, 2013, 14, 448-459.

15. R. A. Shenoi, J. K. Narayanannair, J. L. Hamilton, B. F. L. Lai, S. Horte, R. K. Kainthan, J. P. Varghese, K. G. Rajeev, M. Manoharan and J. N. Kizhakkedathu, Journal of the American Chemical Society, 2012, 134, 14945-14957.

16. S. S. Muller, T. Fritz, M. Gimnich, M. Worm, M. Helm and H. Frey, Polymer Chemistry, 2016, 7, 6257-6268.

17. S. Son, E. Shin and B.-S. Kim, Macromolecules, 2015, 48, 600-609.

18. R. A. Shenoi, I. Chafeeva, B. F. L. Lai, S. Horte and J. N. Kizhakkedathu, Journal of Polymer Science Part A: Polymer Chemistry, 2015, 53, 2104-2115.

19. R. Namgung, Y. Mi Lee, J. Kim, Y. Jang, B.-H. Lee, I.-S. Kim, P. Sokkar, Y. M. Rhee, A. S. Hoffman and W. J. Kim, Nature Communications, 2014, 5, 3702.

20. W. Zhang, Y. Zhang, M. Löbler, K.-P. Schmitz, A. Ahmad, I. Pyykkö and J. Zou, International Journal of Nanomedicine, 2011, 6, 535-546.

21. M. Hu, M. Chen, G. Li, Y. Pang, D. Wang, J. Wu, F. Qiu, X. Zhu and J. Sun, Biomacromolecules, 2012, 13, 3552-3561.

22. C. C. Lee, S. M. Grayson and J. M. J. Frechet, Journal of Polymer Science Part A: Polymer Chemistry, 2004, 42, 3563-3578.

23. A. Parssinen, M. Kohlmayr, M. Leskela, M. Lahcini and T. Repo, Polymer Chemistry, 2010, 1, 834-836.

24. A. R. Spears, J. Waksal, C. McQuade, L. Lanier and E. Harth, Chemical Communications, 2013, 49, 2394-2396.

25. J. Zhou, W. Wang, S. Villarroya, K. J. Thurecht and S. M. Howdle, Chemical Communications, 2008, DOI: 10.1039/b810297j, 5806-5808.

26. F. Du, S. Hönzke, F. Neumann, J. Keilitz, W. Chen, N. Ma, S. Hedtrich and R. Haag, Journal of Controlled Release, 2016, 242, 42-49.

27. F. M. Batz, W. Klipper, H. C. Korting, F. Henkler, R. Landsiedel, A. Luch, U. von Fritschen, G. Weindl and M. Schäfer-Korting, European Journal of Pharmaceutics and Biopharmaceutics, 2013, 84, 374-385.

28. Y. G. Bachhav, K. Mondon, Y. N. Kalia, R. Gurny and M. Möller, Journal of Controlled Release, 2011, 153, 126-132.

29. M. Lapteva, K. Mondon, M. Möller, R. Gurny and Y. N. Kalia, Molecular Pharmaceutics, 2014, 11, 2989-3001.

30. F. Zabihi, S. Wieczorek, M. Dimde, S. Hedtrich, H. G. Börner and R. Haag, Journal of Controlled Release, 2016, 242, 35-41.

31. H. Jun, T. H. Kim, S. W. Han, M. Seo, J. W. Kim and Y. S. Nam, Colloid and Polymer Science, 2015, 293, 2949-2956.

32. Y.-J. Kim, B. Kim, D. C. Hyun, J. W. Kim, H.-E. Shim, S.-W. Kang and U. Jeong, Macromolecular Chemistry and Physics, 2015, 216, 1161-1170.

33. M. Ejaz, A. M. Alb and S. M. Grayson, Reactive and Functional Polymers, 2016, 102, 39-46.

34. M. Adeli, A. Kakanejadifard, M. Khani, F. Bani, R. Kabiri and M. Sadeghizad, Journal of Materials Chemistry B, 2014, 2, 3589-3596.

35. A. Sunder, R. Hanselmann, H. Frey and R. Mülhaupt, Macromolecules, 1999, 32, 4240-4246.

36. A. R. Spears, M. A. Marin, J. R. Montenegro-Burke, B. C. Evans, J. McLean and E. Harth, Macromolecules, 2016, 49, 2022-2027.

37. I. Donskyi, K. Achazi, V. Wycisk, C. Bottcher and M. Adeli, Chemical Communications, 2016, 52, 4373-4376.

38. A. Vogel and H. W. Siesler, Macromolecular Symposia, 2008, 265, 183-194.

39. W. L. Chen, Y. F. Peng, S. K. Chiang and M. H. Huang, International Journal of Nanomedicine, 2015, 10, 2815-2822.

40. R. K. Kainthan, J. Janzen, E. Levin, D. V. Devine and D. E. Brooks, Biomacromolecules, 2006, 7, 703-709.

41. N. Bhaskar, N. Padmavathy, S. Jain, S. Bose and B. Basu, ACS Applied Materials & Interfaces, 2016, 8, 29721-29733.

42. Paulus, F.; Steinhilber, D.; Welker, P.; Mangoldt, D.; Licha, K.; Depner, H.; Sigrist, S.; Haag, R. Polym. Chem. 2014, 5, 5020-5028.

43. Weinhart, M.; Gr, D.; Enders, S.; Dernedde, J.; Haag, R. 2011, 2502-2511.

44. Weinhart, M.; Gro, D.; Enders, S.; Riese, S. B.; Dernedde, J.; Kainthan, R. K.; Brooks, D. E.; Haag, R. 2011, 1088-1098.

45. Pant, K.; Gröger, D.; Bergmann, R.; Pietzsch, J.; Steinbach, J.; Graham, B.; Spiccia, L.; Berthon, F.; Czarny, B.; Devel, L.; Dive, V.; Stephan, H.; Haag, R. Bioconjug. Chem. 2015, 26 (5), 906-918.

46. Riese, S. B.; Dernedde, J.; Reimann, S.; Gröger, D.; Kühne, C.; Riese, S. B.; Dernedde, J..

47. Baumann, K.; Kowalczyk, D.; Kunz, H. Angew. Chemie - Int. Ed. 2008, 47 (18), 3445-3449.

48. S. Enders, G. Bernhard, A. Zakrzewicz, R. Tauber, Biochim. Biophys. Acta, Gen. Subj. 2007, 1770, 1441.

**Claims**

1. Method for manufacturing a hyperbranched polyester polyol derivative, comprising the following steps:

    a) reacting only glycidol and $\varepsilon$-caprolactone to obtain a hyperbranched polyester polyol in which caprolactone residues and glycerol residues are randomly arranged,
    b) reacting the hyperbranched polyester polyol of step a) with a sulfation reagent to obtain a sulfated hyperbranched polyester polyol as hyperbranched polyester polyol derivative.

2. Method according to claim 1, **characterized in that** step a) is carried out in presence of a catalyst.

3. Method according to claim 2, **characterized in that** the catalyst is a Lewis acid.

4. Method according to claim 2 or 3, **characterized in that** the catalyst is tin(II) 2-ethylhexanoate.

5. Method according to any of the preceding claims, **characterized in that** a molar ratio between glycidol and $\epsilon$-caprolactone is between 1:1 and 10:1.

6. Method according to any of the preceding claims, **characterized in that** the sulfation reagent is a sulfur trioxide base complex.

7. Hyperbranched polyester polyol derivative obtainable by a method according to any of the preceding claims.

8. Hyperbranched polyester polyol derivative according to claim 7, **characterized in that** it has a degree of sulfation of from 70 to 100 %.

9. Hyperbranched polyester polyol derivative according to claim 7 or 8 for use in therapy.

10. Hyperbranched polyester polyol derivative according to claim 7 or 8 for use according to claim 9, **characterized in that** the therapy comprises inhibiting the complement system of an organism by the hyperbranched polyester polyol derivative and/or inhibiting L-selectin binding to its natural receptor by the hyperbranched polyester polyol derivative, or treating an inflammatory disease.

11. Hyperbranched polyester polyol derivative according to claim 7 or 8 for use in targeting inflammatory tissue in vivo.

12. Hyperbranched polyester polyol derivative according to claim 7 or 8 for use according to claim 11, **characterized in that** a probe is conjugated to the hyperbranched polyester polyol derivative.

13. Medicament, comprising a hyperbranched polyester polyol derivative according to claim 7 or 8 both as carrier for a pharmaceutically active substance and as a pharmaceutically active substance itself.

14. Hyperbranched polyester polyol obtainable by step a) of the method according to claim 1 for use as carrier for a pharmaceutically active substance.

15. Hyperbranched polyester polyol obtainable by step a) of the method according to claim 1 or hyperbranched polyester polyol derivative according to claim 7 or 8 for use in intradermal delivery of a pharmaceutically active substance.

FIG 1

FIG 2A

FIG 2B

FIG 2C

FIG 2D

FIG 3

FIG 4

FIG 5A

FIG 5B

FIG 6A

FIG 6B

FIG 7

FIG 8

FIG 9

FIG 10

FIG 11

FIG 12

FIG 13

FIG 14

Europäisches
Patentamt

European
Patent Office

Office européen
des brevets

## EUROPEAN SEARCH REPORT

Application Number

EP 17 20 1626

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Melannie S Carna ET AL: "Glycerol-modified poly--caprolactone nanoparticles for drug delivery application", , 31 January 2014 (2014-01-31), pages 38-46, XP055453980, Retrieved from the Internet: URL:http://kimika.pfcs.org.ph/index.php/ki mika/article/view/178 [retrieved on 2018-02-23] | 14,15 | INV. C08G83/00 C08G65/22 C08G65/26 C08G65/326 A61K47/34 C08G63/08 C08G63/85 C08G63/91 C08G65/334 |
| A | * abstract * * page 40, right-hand column, paragraph 2 * ----- | 1-13 | |
| A | LOUIS M. PITET ET AL: "Linear and Branched Architectures from the Polymerization of Lactide with Glycidol", MACROMOLECULES, vol. 40, no. 7, 1 April 2007 (2007-04-01), pages 2327-2334, XP055454102, US ISSN: 0024-9297, DOI: 10.1021/ma0618068 * abstract * * page 2328, right-hand column, paragraph 3 - page 2330, left-hand column, paragraph 1 * ----- | 1-15 | TECHNICAL FIELDS SEARCHED (IPC)  C08G A61K |
| A | WO 2008/015015 A2 (UNIV BERLIN FREIE [DE]; CHARITE UNIVERSITAETSMEDIZIN [DE]; HAAG RAINER) 7 February 2008 (2008-02-07) * claims 1-46 * ----- | 1-15 | |

The present search report has been drawn up for all claims

| Place of search | Date of completion of the search | Examiner |
|---|---|---|
| The Hague | 26 February 2018 | Costantini, Nicola |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 20 1626

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2018

| Patent document cited in search report | | Publication date | Patent family member(s) | | Publication date |
|---|---|---|---|---|---|
| WO 2008015015 | A2 | 07-02-2008 | CA | 2659940 A1 | 07-02-2008 |
| | | | CN | 101506272 A | 12-08-2009 |
| | | | CN | 102585202 A | 18-07-2012 |
| | | | DE | 102006036326 A1 | 07-02-2008 |
| | | | EP | 2046868 A2 | 15-04-2009 |
| | | | JP | 2009545545 A | 24-12-2009 |
| | | | JP | 2013166756 A | 29-08-2013 |
| | | | US | 2012328519 A1 | 27-12-2012 |
| | | | US | 2014363507 A1 | 11-12-2014 |
| | | | WO | 2008015015 A2 | 07-02-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012031245 A1
- US 20150037375 A1
- US 20160331875 A1

### Non-patent literature cited in the description

- **LI, Z. ; LI, J.** Control of hyperbranched structure of polycaprolactone/poly (ethylene glycol) polyurethane block copolymers by glycerol and their hydrogels for potential cell delivery. *The Journal of Physical Chemistry B,* 2013, vol. 117 (47), 14763-14774
- **ZHOU, J. ; WANG, W. ; VILLARROYA, S. ; THURECHT, K. J. ; HOWDLE, S. M.** Epoxy functionalised poly (ε-caprolactone): synthesis and application. *Chemical Communications,* 2008, vol. 44, 5806-5808
- **F. DU ; S. HÖNZKE ; F. NEUMANN ; J. KEILITZ ; W. CHEN ; N. MA ; S. HEDTRICH ; R. HAAG.** *Journal of Controlled Release,* 2016, vol. 242, 42-49
- **F. M. BÄTZ ; W. KLIPPER ; H. C. KORTING ; F. HENKLER ; R. LANDSIEDEL ; A. LUCH ; U. VON FRITSCHEN ; G. WEINDL ; M. SCHAFER-KORTING.** *European Journal of Pharmaceutics and Biopharmaceutics,* 2013, vol. 84, 374-385
- **Y. G. BACHHAV ; K. MONDON ; Y. N. KALIA ; R. GURNY ; M. MÖLLER.** *Journal of Controlled Release,* 2011, vol. 153, 126-132
- **M. LAPTEVA ; K. MONDON ; M. MÖLLER ; R. GURNY ; Y. N. KALIA.** *Molecular Pharmaceutics,* 2014, vol. 11, 2989-3001
- **F. ZABIHI ; S. WIECZOREK ; M. DIMDE ; S. HEDTRICH ; H. G. BÖRNER ; R. HAAG.** *Journal of Controlled Release,* 2016, vol. 242, 35-41
- **A. SUNDER ; R. HANSELMANN ; H. FREY ; R. MÜLHAUPT.** *Macromolecules,* 1999, vol. 32, 4240-4246
- **S. ENDERS ; G. BERNHARD ; A. ZAKRZEWICZ ; R. TAUBER.** Biochim. Biophys. Acta. *Gen. Subj.,* 2007, vol. 1770, 1441
- **Z. LI ; J. LI.** *The Journal of Physical Chemistry B,* 2013, vol. 117, 14763-14774
- **D. WILMS ; S.-E. STIRIBA ; H. FREY.** *Accounts of Chemical Research,* 2010, vol. 43, 129-141
- **I. GITSOV.** *Journal of Polymer Science Part A: Polymer Chemistry,* 2008, vol. 46, 5295-5314
- **E. MOHAMMADIFAR ; A. NEMATI KHARAT ; M. ADELI.** *Journal of Materials Chemistry B,* 2015, vol. 3, 3896-3921
- **N. KURNIASIH ; J. KEILITZ ; R. HAAG.** *Chemical Society Reviews,* 2015, vol. 44, 4145-4164
- **S. STEFANI ; I. N. KURNIASIH ; S. K. SHARMA ; C. BOTTCHER ; P. SERVIN ; R. HAAG.** *Polymer Chemistry,* 2016, vol. 7, 887-898
- **R. A. SHENOI ; B. F. L. LAI ; J. N. KIZHAKKEDATHU.** *Biomacromolecules,* 2012, vol. 13, 3018-3030
- **R. K. KAINTHAN ; E. B. MULIAWAN ; S. G. HATZIKIRIAKOS ; D. E. BROOKS.** *Macromolecules,* 2006, vol. 39, 7708-7717
- **R. K. KAINTHAN ; D. E. BROOKS.** *Biomaterials,* 2007, vol. 28, 4779-4787
- **E. MOHAMMADIFAR ; A. BODAGHI ; A. DADKHAHTEHRANI ; A. NEMATI KHARAT ; M. ADELI ; R. HAAG.** *ACS Macro Letters,* 2016, 35-40
- **C. DINGELS ; S. S. MÜLLER ; T. STEINBACH ; C. TONHAUSER ; H. FREY.** *Biomacromolecules,* 2013, vol. 14, 448-459
- **R. A. SHENOI ; J. K. NARAYANANNAIR ; J. L. HAMILTON ; B. F. L. LAI ; S. HORTE ; R. K. KAINTHAN ; J. P. VARGHESE ; K. G. RAJEEV ; M. MANOHARAN ; J. N. KIZHAKKEDATHU.** *Journal of the American Chemical Society,* 2012, vol. 134, 14945-14957
- **S. S. MULLER ; T. FRITZ ; M. GIMNICH ; M. WORM ; M. HELM ; H. FREY.** *Polymer Chemistry,* 2016, vol. 7, 6257-6268
- **S. SON ; E. SHIN ; B.-S. KIM.** *Macromolecules,* 2015, vol. 48, 600-609
- **R. A. SHENOI ; I. CHAFEEVA ; B. F. L. LAI ; S. HORTE ; J. N. KIZHAKKEDATHU.** *Journal of Polymer Science Part A: Polymer Chemistry,* 2015, vol. 53, 2104-2115
- **R. NAMGUNG ; Y. MI LEE ; J. KIM ; Y. JANG ; B.-H. LEE ; I.-S. KIM ; P. SOKKAR ; Y. M. RHEE ; A. S. HOFFMAN ; W. J. KIM.** *Nature Communications,* 2014, vol. 5, 3702

- W. ZHANG ; Y. ZHANG ; M. LÖBLER ; K.-P. SCHMITZ ; A. AHMAD ; I. PYYKKÖ ; J. ZOU. *International Journal of Nanomedicine,* 2011, vol. 6, 535-546
- M. HU ; M. CHEN ; G. LI ; Y. PANG ; D. WANG ; J. WU ; F. QIU ; X. ZHU ; J. SUN. *Biomacromolecules,* 2012, vol. 13, 3552-3561
- C. C. LEE ; S. M. GRAYSON ; J. M. J. FRECHET. *Journal of Polymer Science Part A: Polymer Chemistry,* 2004, vol. 42, 3563-3578
- A. PARSSINEN ; M. KOHLMAYR ; M. LESKELA ; M. LAHCINI ; T. REPO. *Polymer Chemistry,* 2010, vol. 1, 834-836
- A. R. SPEARS ; J. WAKSAL ; C. MCQUADE ; L. LANIER ; E. HARTH. *Chemical Communications,* 2013, vol. 49, 2394-2396
- J. ZHOU ; W. WANG ; S. VILLARROYA ; K. J. THURECHT ; S. M. HOWDLE. *Chemical Communications,* 2008, 5806-5808
- F. M. BATZ ; W. KLIPPER ; H. C. KORTING ; F. HENKLER ; R. LANDSIEDEL ; A. LUCH ; U. VON FRITSCHEN ; G. WEINDL ; M. SCHÄFER-KORTING. *European Journal of Pharmaceutics and Biopharmaceutics,* 2013, vol. 84, 374-385
- H. JUN ; T. H. KIM ; S. W. HAN ; M. SEO ; J. W. KIM ; Y. S. NAM. *Colloid and Polymer Science,* 2015, vol. 293, 2949-2956
- Y.-J. KIM ; B. KIM ; D. C. HYUN ; J. W. KIM ; H.-E. SHIM ; S.-W. KANG ; U. JEONG. *Macromolecular Chemistry and Physics,* 2015, vol. 216, 1161-1170
- M. EJAZ ; A. M. ALB ; S. M. GRAYSON. *Reactive and Functional Polymers,* 2016, vol. 102, 39-46
- M. ADELI ; A. KAKANEJADIFARD ; M. KHANI ; F. BANI ; R. KABIRI ; M. SADEGHIZAD. *Journal of Materials Chemistry B,* 2014, vol. 2, 3589-3596
- A. R. SPEARS ; M. A. MARIN ; J. R. MONTENEGRO-BURKE ; B. C. EVANS ; J. MCLEAN ; E. HARTH. *Macromolecules,* 2016, vol. 49, 2022-2027
- I. DONSKYI ; K. ACHAZI ; V. WYCISK ; C. BOTTCHER ; M. ADELI. *Chemical Communications,* 2016, vol. 52, 4373-4376
- A. VOGEL ; H. W. SIESLER. *Macromolecular Symposia,* 2008, vol. 265, 183-194
- W. L. CHEN ; Y. F. PENG ; S. K. CHIANG ; M. H. HUANG. *International Journal of Nanomedicine,* 2015, vol. 10, 2815-2822
- R. K. KAINTHAN ; J. JANZEN ; E. LEVIN ; D. V. DEVINE ; D. E. BROOKS. *Biomacromolecules,* 2006, vol. 7, 703-709
- N. BHASKAR ; N. PADMAVATHY ; S. JAIN ; S. BOSE ; B. BASU. *ACS Applied Materials & Interfaces,* 2016, vol. 8, 29721-29733
- PAULUS, F. ; STEINHILBER, D. ; WELKER, P. ; MANGOLDT, D. ; LICHA, K. ; DEPNER, H. ; SIGRIST, S. ; HAAG, R. *Polym. Chem.,* 2014, vol. 5, 5020-5028
- PANT, K. ; GRÖGER, D. ; BERGMANN, R. ; PIETZSCH, J. ; STEINBACH, J. ; GRAHAM, B. ; SPICCIA, L. ; BERTHON, F. ; CZARNY, B. ; DEVEL, L. *Bioconjug. Chem.,* 2015, vol. 26 (5), 609-918
- BAUMANN, K. ; KOWALCZYK, D. ; KUNZ, H. *Angew. Chemie - Int. Ed.,* 2008, vol. 47 (18), 3445-3449
- S. ENDERS ; G. BERNHARD ; A. ZAKRZEWICZ ; R. TAUBER. *Biochim. Biophys. Acta, Gen. Subj.,* 2007, vol. 1770, 1441